# EUROPÄISCHE PATENTSCHRIFT

(11) **EP 0 684 251 B1**
(45) Veröffentlichungstag und Bekanntmachung des Hinweises auf die Patenterteilung: **18.08.1999**
(21) Anmeldenummer: 95106072.2
(22) Anmeldetag: 24.04.1995
(51) Int. Cl.: C07H 15/04, B01J 23/85, B01J 23/86, B01J 23/88

(54) **Verfahren zur Herstellung von alpha-D-Glucopyranosido-1,6-mannit und -sorbit aus alpha-D-Glucopyranosido-1,6-fructose**
Process for the preparation of alpha-D-glucopyranosido-1,6-mannit and -sorbit from alpha-D-glucopyranisido-1,6-fructose
Procédé pour la préparation d'alpha-D-glucopyranisido-1,6-mannite et alpha-D-glucopyraniside-1,6-sorbite à partir de alpha-D-glucopyranisido-1,6-fructose

(30) Priorität: 06.05.1994 DE 4416115
(43) Veröffentlichungstag der Anmeldung: 29.11.1995
(73) Patentinhaber: BAYER AG, 51368 Leverkusen (DE)
(72) Erfinder: Darsow, Gerhard, Dr., D-47804 Krefeld (DE)

(56) Entgegenhaltungen:
- EP-A- 0 152 779
- EP-A- 0 423 525
- DE-A- 2 520 173
- DE-A- 4 026 351
- DE-C- 554 074
- DATABASE WPI, Woche 8017, Derwent Publications Ltd., London, GB, AN 80-30077C, Klasse , JP 55035064

## Beschreibung

Die Erfindung betrifft ein kostengünstiges Verfahren zur Herstellung eines Gemischs der diastereomeren Zuckeralkohole α-D-Glucopyranosido-1,6-mannit und α-D-Glucopyranosido-1,6-sorbit aus α-D-Glucopyranosido-1,6-fructose durch kontinuierliche katalytische Hydrierung mit Wasserstoff.

Der Reaktionsverlauf läßt sich durch das folgende Reaktionsschema veranschaulichen:

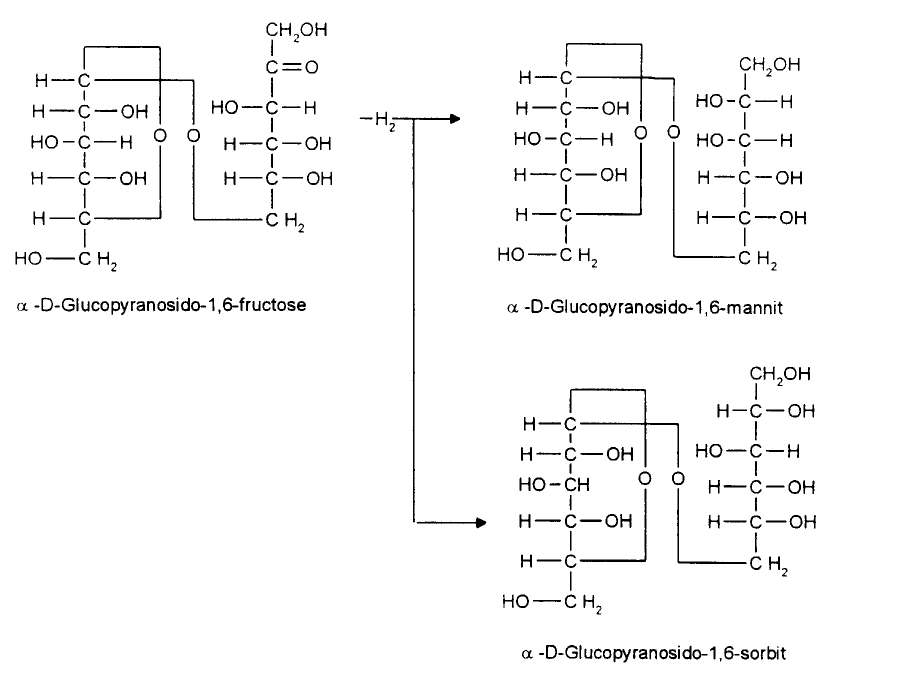

Bei den bekannten Verfahren zur Herstellung von α-D-Glucopyranosido-1,6-sorbit (DE-PS 2 217 628) und α-D-Glucopyranosido-1,6-mannit (DE-AS 2 520 173) wird im diskontinuierlichen Suspensionsverfahren (Batch-process) als Hydrierkatalysator jeweils ein pulverförmiger Nickelkatalysator eingesetzt. Diskontinuierliche Verfahren haben den Nachteil, daß ihre Kapazität relativ zum Reaktionsvolumen sehr klein ist und somit ein Bedarf nach großen Reaktionsapparaturen und Lagertanks besteht. Energieverbrauch und Personalbedarf sind verhältnismäßig hoch. Kontinuierliche Pulverkatalysatorverfahren, die mit mehreren in Kaskade geschalteten Hydrierreaktoren arbeiten, vermeiden einen Teil dieser Nachteile. Es bleibt jedoch das Erfordernis, den pulverförmigen Katalysator gezielt zu dotieren, umzupumpen und quantitativ vom Reaktionsprodukt abzufiltrieren. Die Katalysatorschlammpumpen unterliegen einem hohen mechanischen Verschleiß. Die quantitative Entfernung des pulverförmigen Katalysators aus dem Reaktionsprodukt ist aufwendig. Ferner ist die Gefahr groß, die Katalysatoraktivität durch die zusätzlichen Operationen verhältnismäßig schnell zu verringern. Es ist daher vorteilhaft, die Reaktion über einen fest angeordneten Katalysator ablaufen zu lassen. Ein solcher Katalysator muß eine hohe Aktivität besitzen, die über einen längeren Zeitraum nicht nachlassen darf, weil häufige Katalysatorwechsel bei Festbettreaktionen ebenfalls aufwendig sind.

Aus der EP-A 152 779 ist ein Verfahren zur kontinuierlichen Hydrierung von Zuckern zu den entsprechenden Zuckeralkoholen über trägerfreien Formkörpern von Elementen der 8. Nebengruppe des Periodensystems bekannt, wobei diese trägerfreien Formkörper vorzugsweise durch Verpressen und/oder Verkleben von Metallpulver hergestellt worden sind. Das Verfahren liefert die gewünschten Produkte in ausgezeichneter Reinheit in sehr guten Ausbeuten. Es wäre allerdings wünschenswert, die hohen Katalysatorkosten zu senken. Außerdem ist man immer bestrebt, ein Verfahren bei möglichst niedriger Temperatur durchzuführen, um die Energiekosten zu senken.

Es wurde überraschenderweise gefunden, daß Elemente der VI. Nebengruppe des Periodensystems enthaltende Metallpulver von Nickel, Kobalt und Eisen oder ihrer Legierungen nach ihrer Verpressung zu Formkörpern nicht nur ebensogut die Hydrierung von α-D-Glucopyranosido-1,6-fructose zu einem Gemisch von α-D-Glucopyranosido-1,6-mannit und α-D-Glucopyranosido-1,6-sorbit katalysieren, sondern daß Katalysatoren aus diesen Metallen oder Metall-Legierungen, die um 30 - 45 % preiswerter sind, sogar eine erheblich höhere Hydrieraktivität aufweisen, so daß die Hydrierreaktion bei einer bis zu 30°C niedrigeren Reaktionstemperatur durchgeführt werden kann. Dabei können die zum Einsatz kommenden Pulver zusätzlich gewisse Anteile anderer nicht katalytisch wirkender Metalle (z.B. Mangan, Silicium, Aluminium, Titan) enthalten, ohne daß die hohe Aktivität gemindert wird.

Gegenstand der Erfindung ist daher ein Verfahren zur Herstellung eines Gemisches von α-D-Glucopyranosido-1,6-mannit und α-D-Glucopyranosido-1,6-sorbit aus α-D-Glucopyranosido-1,6-fructose durch katalytische Hydrierung in wäßriger Lösung mit Wasserstoff unter erhöhtem Druck und bei erhöhter Temperatur, dadurch gekennzeichnet, daß man die Hydrierung kontinuierlich bei einem Wasserstoffdruck von 100 bis 400 bar und Temperaturen von 40 bis 80°C im Festbettverfahren über als Hydrierungskatalysatoren dienenden trägerfreien Formkörpern mit einer Druckfestigkeit von 20 bis 250 N und einer inneren Oberfläche von 10 bis 80 m²/g von
(i) einem oder mehreren Elementen der Eisenuntergruppe der VIII. Nebengruppe des Periodensystems durchführt, die zusätzlich mit
(ii) aktivierend wirkenden Elementen der VI. Nebengruppe legiert sind.

Die Druckfestigkeit der trägerfreien Formkörper kann nach DIN 50 106 bestimmt werden.

Die Überprüfung von trägerfreien Formkörpern auf die anspruchsgemäßen inneren Oberflächen und damit auf Brauchbarkeit für das erfindungsgemäße Verfahren können nach Methoden durchgeführt werden, die von F. M. Nelsen und F. T. Eggertsen, Analyt. Chem. 30 (1958), 1387 bzw. S. J. Gregg und S. W. Sing, Adsorption, Surface Area and Porosity, London 1967, Kap. 2 und 8, beschrieben worden sind.

Die Eisenuntergruppe der VIII. Nebengruppe des Periodensystems enthält die Elemente Eisen, Cobalt und Nickel. Die erfindungsgemäß zu verwendenden trägerfreien Formkörper enthalten eines oder mehrere dieser Metalle in Mengen von mindestens 60, vorzugsweise mindestens 70, insbesondere mindestens 80 Gew.-%, bezogen auf trägerfreie Formkörper.

Die VI. Nebengruppe des Periodensystems enthält die Elemente Chrom, Molybdän und Wolfram. Die erfindungsgemäß zu verwendenden trägerfreien Formkörper enthalten eines oder mehrere dieser Metalle in Mengen von mindestens 0,1, vorzugsweise mindestens 0,3, insbesondere mindestens 0,5 Gew.-%, bezogen auf trägerfreie Formkörper; sie enthalten eines oder mehrere dieser Metalle in Mengen von höchstens 20, vorzugsweise höchstens 10 und insbesondere höchstens 5 Gew.-%, bezogen auf trägerfreie Formkörper.

Die erfindungsgemäß zu verwendenden trägerfreien Formkörper können darüber hinaus - jeweils bezogen auf trägerfreie Formkörper - bis zu 20, vorzugsweise bis zu 15, insbesondere bis zu 10 Gew.-% anderer Metalle enthalten; Beispiele solcher Metalle, die nicht katalytisch wirken müssen, umfassen Aluminium, Silicium, Titan und Mangan. Nach einer bevorzugten Ausführungsform enthalten die trägerfreien Formkörper außer den Komponenten (i) und (ii) nicht mehr als 10 Gew.-% Aluminium und nicht mehr als 5 Gew.-% anderer Metalle.

Nach dem erfindungsgemäßen Verfahren ist es möglich, das kristallisierte Gemisch der beiden diastereomeren Zuckeralkohole in einer Reinheit von über 99 Gew.-% herzustellen, wobei der Gehalt an nicht umgesetzter α-D-Glucopyranosido-1,6-fructose unter ≤ 0,2 Gew.-% und die Summe von Sorbit und Mannit unter 0,3 Gew.-% liegen. Als Ausgangsverbindung für das erfindungsgemäße Verfahren wird kristalline α-D-Glucopyranosido-1,6-fructose eingesetzt. Diese Substanz kann aus Saccharose-Lösungen durch enzymatische Umwandlung mit lebenden oder immobilisierten Zellsystemen nach bekannten Methoden (z.B. DE-PS 1 049 800) hergestellt werden.

Die α-D-Glucopyranosido-1,6-fructose wird am besten in sauerstoff-freiem, entionisiertem Wasser gelöst. Nach einer bevorzugten Ausführungsform geht man folgendermaßen vor:

Aus α-D-Glucopyranosido-1,6-fructose und entionisiertem Trinkwasser wird eine 45- bis 60 gew.-%ige, bevorzugt 50- bis 55 gew.-%ige, wäßrige Lösung hergestellt, deren pH-Wert auf 3,5 - 10,5 bevorzugt 5 - 6,5, eingestellt wird. Kristalline α-D-Glucopyranosido-1,6-fructose zeigt, gelöst in Wasser mit einem pH-Wert von 7, entweder eine neutrale oder - bedingt durch eine möglicherweise durch Cannizarro-Reaktion hervorgerufene spurenweise Gluconsäurebildung - eine schwach saure Reaktion. Die gewünschte pH-Wert-Einstellung kann z.B. durch Zugabe einer möglichst reinen organischen Säure, wie z.B. Zitronensäure, Sorbinsäure sowie Zuckersäuren, erfolgen.

Für den Hydrierprozeß wird auf einen Druck von 100 bis 400 bar, bevorzugt 200 bis 300 bar, vorkomprimierter reiner Wasserstoff eingesetzt. Die Hydrierung erfolgt kontinuierlich im Festbettverfahren an den als Hydrierkatalysatoren dienenden trägerfreien Formkörpern metallischer Art, indem man die zu hydrierende Lösung entweder im Gleichstrom mit dem zuvor zugemischten Wasserstoff von unten oder oben kommend über die in einen Hydrierreaktor gefüllten Formkörper strömen läßt oder auch von unten kommend dem von oben einströmenden Wasserstoff entgegenführt oder umgekehrt (Gegenstromverfahren).

Der Hydrierreaktor kann entweder ein einzelnes Hochdruckrohr aus Stahl oder einer Stahllegierung sein, das mit dem trägerfreien Formkörper ganz oder teilweise gefüllt wird, wobei auch die Anwendung auf Horden (Drahtkörbe o. ä.) nützlich sein kann, oder aber ein ummanteltes Hochdruckrohrbündel, dessen Einzelrohre mit Formkörpern ganz oder teilweise gefüllt werden.

Die Herstellung der trägerfreien Formkörper kann nach gebräuchlichen Methoden durch Verpressen der Metallpulver auf Tablettier- oder Pelletiermaschinen unter hohem Druck erfolgen, wobei zur Verbesserung des Haftvermögens der Metallpartikel auch Graphit in Mengen von 0,5 - 1,5 Gew.-%, bezogen auf das Gesamtgewicht der den Katalysator bildenden Bestandteile, oder Klebstoffe in kleinen Mengen zum Einsatz kommen können. Die Herstellung der trägerfreien Formkörper wird vorzugsweise in einer sauerstoff-freien Atmosphäre erfolgen, um Oberflächenoxidationen zu vermeiden. Am wirksamsten und für die Reaktionsführung am günstigsten sind tablettierte oder pelletierte Formkörper mit Durchmessern von 3 bis 7 mm. Von erheblicher Bedeutung ist die Druckfestigkeit der Formkörper, die erfindungsgemäß bei Werten von 20 bis 250 N, bevorzugt 110 bis 220 N, liegt. Niedrigere Druckfestigkeiten führen zu Formkörperzerfall bzw. erosivem Abrieb, was eine metallische Kontaminierung des Reaktionsproduktes bewirken würde. Von erheblicher Bedeutung ist weiterhin die innere Oberfläche der Formkörper, die erfindungsgemäß bei Werten von 10 bis 80 m²/g liegt und ausschlaggebend für einen möglichst quantitativen Umsatz der Einsatzstoffe ist.

Der Hydrierprozeß wird bei einer Temperatur von 40 bis 80°C, bevorzugt 55 bis 70°C, durchgeführt. Niedrigere Temperaturen bedingen höhere Verweilzeiten oder Verzicht auf einen quantitativen Umsatz der α-D-Glucopyranosido-1,6-fructose. Höhere Temperaturen führen zu vermehrter Bildung von Zuckeralkoholen von Monosacchariden (Sorbit bzw. Mannit) sowie zu unkontrollierten Nebenreaktionen (Karamelisierung, hydrierende Crackung), was zu Verfärbungen sowie zur Bildung weiterer unerwünschter Nebenprodukte führen kann. Die stündliche Katalysatorbelastung kann 45 bis 60 g α-D-Glucopyranosido-1,6-fructose/l Katalysator, bevorzugt 50 bis 55 g/l betragen. Bei den geschilderten Reaktionsbedingungen sind ganz unerwartet hohe Katalysatorstandzeiten von 15.000 Stunden und mehr zu erzielen, was zu bisher bei der Hydrierung von α-D-Glucopyranosido-1,6-fructose noch nicht erreichten Katalysatorverbräuchen von ≤ 0,12 Gew.-% führt. Damit liegen die hauptsächlichen technischen Vorteile des erfindungsgemäßen Verfahrens, außer in den hohen Ausbeuten an α-D-Glucopyranosido-1,6-mannit und α-D-Glucopyranosido-1,6-sorbit und der Reinheit des Gemisches, die keine weiteren Reinigungsprozeduren notwendig macht, besonders in den niedrigen Katalysatorkosten.

Die den Reaktor verlassende hydrierte wäßrige Lösung, die die beiden Zuckeralkohole α-D-Glucopyranosido-1,6-mannit und α-D-Glucopyranosido-1,6-sorbit im Verhältnis von ca. 1 : 1 enthält, wird nach der Entspannung, bei welcher man den überschüssigen Wasserstoff abfangen und nach erfolgter Komprimierung erneut zum Einsatz bringen kann, filtriert und kann so bereits direkt als Zuckeraustauschstoffmischung in flüssiger Form zum Einsatz kommen.

Das Wasser der Lösung läßt sich aber auch auf bekannte Weise über Sprühtrockner, Trockenwalzen oder durch Gefriertrocknung entfernen. Im Regelfall wird die nach dem Filtrieren erhaltene farblose und glasklare Lösung in einem Fallfilmverdampfer oder einem ähnlich arbeitenden Gerät auf einen Zuckeralkoholgehalt von ca. 80 Gew.-% aufkonzentriert und anschließend in einem Vakuum-Kristallisationsgerät zur vollständigen Kristallisation gebracht. Das Kristallisat läßt sich durch einen nachgeschalteten Mahlprozeß und eventuelle Siebung auf eine einheitliche Korngröße bringen. Obwohl das so gewonnene Produkt rieselfähig ist und völlig trocken erscheint, hat es einen Kristallwassergehalt von ca. 5 Gew.-%, was darauf zurückzuführen ist, daß α-D-Glucopyranosido-1,6-mannit im Gegensatz zu α-D-Glucopyranosido-1,6-sorbit mit einem Gehalt von etwa 10 Gew.-% Kristallwasser kristallisiert.

Das gewonnene Produkt beginnt bei 90°C zu schmelzen. Eine klare Schmelze bildet sich bei 140°C. Den exakten Schmelzbereich des wasserfreien Substanzgemisches erhält man, wenn man z.B. das wasserhaltige Produkt in einem evakuierbaren Trockengerät bei 110°C und 10 mbar zur Schmelze bringt und aus der Schmelze das Wasser quantitativ verdampfen läßt. Eine so behandelte rekristallisierte Probe zeigt ein Schmelzintervall von 138 bis 143°C.

Die beiden erfindungsgemäß herstellbaren Zuckeralkohole sind bekannt. Sie werden als Einzelstoffe wie als Mischung aufgrund ihres angenehmen süßen Geschmacks, der im Gegensatz zu manchen anderen Zuckeralkoholen frei von Bei- oder Nebengeschmack ist, als kalorienarme Zuckerersatzstoffe eingesetzt, die auch für Diabetiker geeignet und weniger kariogen sind als Saccharose.

In seinem Lösungsverhalten in Wasser liegt das Gemisch von α-D-Glucopyranosido-1,6-mannit und α-D-Glucopyranosido-1,6-sorbit im Temperaturbereich von 0 - 70°C zwischen dem der Reinsubstanzen. Bei Temperaturen über 70°C übertrifft die Löslichkeit des Gemisches die der Reinsubstanzen, was den Einsatz der Mischung als Süßungsmittel für Getränke und Nahrungsmittel immer dann besonders vorteilhaft erscheinen laßt, wenn die infrage kommenden Stoffe stärker gesüßt werden sollen. Sowohl die Einzelverbindungen wie auch das Gemisch zeigen eine Süßkraft, die etwa 45 % der Süßkraft von Saccharose entspricht. Zur Erhöhung der Süßkraft des Gemisches von α-D-Glucopyranosido-1,6-mannit und α-D-Glucopyranosido-1,6-sorbit kann die wäßrige Lösung mit künstlichen Süßstoffen, z.B. Cyclohexylsulfamat oder Asparaginsäure-phenylalanin-methylester, versetzt und durch gemeinsame Vakuumkristallisation in kristalliner Form gewonnen werden. Man kann aber auch die künstlichen Süßstoffe in fester Form mit dem Kristallisat vermischen. Das Gemisch von α-D-Glucopyranosido-1,6-mannit und α-D-Glucopyranosido-1,6-sorbit kann auch in flüssiger oder fester Form mit anderen süßschmeckenden Zuckeralkoholen, z.B. Sorbit, Xylit, Mannit und Lactit vermischt werden. Wegen seiner hohen Karamelisierungstemperatur ist das Gemisch besonders gut als süß schmeckender, struktur- und körperbildender Füllstoff für Schokolade, Pralinen und Gebäck geeignet.

Die Prozentangaben der nachfolgenden Beispiele beziehen sich jeweils auf das Gewicht.

### Beispiele

### Beispiel 1

Ein senkrecht stehendes, wärmeisoliertes Hochdruckrohr aus nichtrostendem Stahl von 45 mm Innendurchmesser und 1 m Länge wird mit 1,4 l eines durch Tablettierung von Pulver einer Ni/Mo-Legierung mit einem Mo-Gehalt von 1,75 % hergestellten Hydrierungskatalysators gefüllt, der bei einer Zylinderhöhe von 5 mm und einem Durchmesser von 5 mm eine Druckfestigkeit von 191 N und eine innere Oberfläche von 58 m²/g aufweist. Durch dieses Rohr werden gemeinsam mit der dreifach molaren Menge von unter einem Druck von 300 bar stehendem hochreinem Wasserstoff stündlich 140 ml einer 50 %igen Lösung von α-D-Glucopyranosido-1,6-fructose in entionisiertem Trinkwasser, die auf einen pH-Wert von 6,0 eingestellt wurde, kontinuierlich gepumpt und zwar von unten nach oben aufsteigend.

Wäßrige Lösung und Wasserstoff werden durch einen Wärmeaustauscher geführt und so hoch erhitzt, daß sie in das Hochdruckrohr mit einer Temperatur von 60°C eintreten. Das das Hochdruckrohr verlassende Gemisch von wäßriger Lösung und überschüssigem Wasserstoff wird über einen Kühler in einen Abscheider geführt, von wo der Wasserstoff nach Ersatz der verbrauchten Menge wieder zusammen mit noch nicht hydrierter Lösung in den Vorwärmer und von da erneut in das Hochdruckrohr gepumpt wird.

Die klare wäßrige Lösung wird entspannt, über ein Feinfilter filtriert, in einem Fallfilmverdampfer auf einen Zuckeralkoholgehalt von ca. 80 % aufkonzentriert und anschließend in einem Vakuumkristaller zur vollständigen Kristallisation gebracht. Das erhaltene feine Kristallpulver besteht aus einem Gemisch aus α-D-Glucopyranosido-1,6-mannit und α-D-Glucopyranosido-1,6-sorbit im Verhältnis 1 : 1 i. T. Der Wassergehalt liegt bei 5 %. Das Gemisch der beiden stereoisomeren Zuckeralkohole ist ansonsten hochrein (Reinheitsgrad ≤ 99,1 %). Der Gehalt an nicht hydrierter α-D-Glucopyranosido-1,6-fructose liegt bei ≤ 0,2 %. Der Gehalt an Sorbit liegt bei ≤ 0,20 %. Mannit konnte nicht nachgewiesen werden. Der Katalysator war auch nach einer Laufzeit von 3.200 Stunden unverändert wirksam.

### Beispiel 2

Durch ein Hochdruckrohr wie im Beispiel 1, aber aus Hochdruckstahl N 9, wird bei einer Temperatur von 65°C und einem Wasserstoffdruck von 200 bar im umgekehrten Reaktionsfluß, wie in Beispiel 1 beschrieben, stündlich eine gleichgroße Menge 50 %iger wäßriger Lösung von α-D-Glucopyranosido-1,6-fructose hydriert, die einen pH-Wert von 5,5 aufweist. Der Katalysator wurde durch Tablettierung von Pulver einer Ni/Mo-Legierung mit einem Mo-Gehalt von 1,62 % und einem Al-Gehalt von 6,1 % hergestellt. Die Tabletten haben bei einer Zylinderhöhe von 5 mm und einem Durchmesser von 5 mm eine Druckfestigkeit von 210 N und eine innere Oberfläche von 71 m²/g.

Nach einer Laufzeit von 1.200 Stunden mit unverminderter Wirksamkeit liegt der Gehalt an α-D-Glucopyranosido-1,6-mannit und α-D-Glucopyranosido-1,6-sorbit des in einem Rotationsverdampfer zur Trocknung eingedampften Reaktionsgemisches bei 99,1 %. Der Gehalt an nicht hydrierter α-D-Glucopyranosido-1,6-fructose liegt bei 0,2 %. Der Gehalt an Sorbit liegt bei 0,2 %. Der Gehalt an Mannit beträgt 0,03 %.

### Beispiel 3

In einem Hochdruckrohr wie in Beispiel 1 wird bei einer Temperatur von 55°C und einem Wasserstoffdruck von 300 bar in gleicher Weise wie in Beispiel 1 stündlich eine gleichgroße Menge einer 55 %igen wäßrigen Lösung von α-D-Glucopyranosido-1,6-fructose hydriert, die einen pH-Wert von 6,5 aufweist. Der Katalysator wurde durch Tablettierung einer pulverisierten Ni/Fe/Mo-Legierung gewonnen. Die Legierung enthält einen Fe-Anteil in Ni von 15 % sowie einen Mo-Gehalt von 1,4 %. Die Tabletten haben bei einer Zylinderhöhe von 3 mm und einem Durchmesser von 3 mm eine Druckfestigkeit von 162 N und eine Oberfläche von 68 m²/g. Das in einem Vakuumkristaller gewonnene 1 : 1-Gemisch von α-D-Glucopyranosido-1,6-mannit und α-D-Glucopyranosido-1,6-sorbit hat einen Reinheitsgrad von ≥ 99,1 %. Der Gehalt an nicht umgesetzter α-D-Glucopyranosido-1,6-fructose liegt bei 0,1 %. Der Sorbit-Gehalt beträgt 0,2 %. Der Mannitanteil beträgt 0,08 %. Der Katalysator war nach einer Laufzeit von 1.100 Stunden noch unverändert wirksam.

### Beispiel 4

In einem Hochdruckrohr wie in Beispiel 1 wird bei einer Temperatur von 65°C und einem Wasserstoffdruck von 200 bar in gleicher Weise wie in Beispiel 1 eine ebenso große Menge einer 45 %igen wäßrigen Lösung von α-D-Glucopyranosido-1,6-fructose hydriert, die einen pH-Wert von 5,5 aufweist. Der Katalysator wurde durch Tablettierung einer pulverisierten Ni/Mo-Legierung mit einem Mo-Gehalt von 0,55 % gewonnen. Die Tabletten haben bei einer Zylinderhöhe von 5 mm und einem Durchmesser von 5 mm eine Druckfestigkeit von 147 N und eine innere Oberfläche von 44 m²/g. Das in einem Vakuum-Drehrohr gewonnene Gemisch von α-D-Glucopyranosido-1,6-mannit und α-D-Glucopyranosido-1,6-sorbit hat einen Reinheitsgrad von 99,4 %. Der Gehalt an nicht umgesetzter α-D-Glucopyranosido-1,6-fructose liegt bei 0,2 %. Der Sorbit-Gehalt beträgt 0,25 %. Mannit läßt sich nicht nachweisen. Der Katalysator war nach einer Laufzeit von 900 Stunden noch unvermindert wirksam.

### Beispiel 5

In einem Hochdruckrohr wie in Beispiel 1 wird bei einer Temperatur von 70°C und einem Wasserstoffdruck von 300 bar in gleicher Weise wie in Beispiel 1 stündlich eine gleichgroße Menge einer 45 %igen wäßrigen Lösung von α-D-Glucopyranosido-1,6-fructose hydriert, die einen pH-Wert von 6,5 aufweist. Der Katalysator wurde durch Tablettierung einer pulverisierten Ni/Mo/Al/Si-Legierung mit einem Mo-Gehalt von 0,35 %, einem Al-Gehalt von 5,4 % und einem Si-Gehalt von 0,4 % gewonnen. Die Tabletten haben bei einer Zylinderhöhe von 5 mm und einem Durchmesser von 5 mm eine Druckfestigkeit von 148 N und eine innere Oberfläche von 61 m²/g. Das in einem Vakuumkristaller gewonnene 1 : 1-Gemisch von α-D-Glucopyranosido-1,6-mannit und α-D-Glucopyranosido-1,6-sorbit hat einen Reinheitsgrad von 99,1 %. Der Gehalt an nicht umgesetzter α-D-Glucopyranosido-1,6-fructose liegt bei 0,2 %. Der Sorbit-Gehalt beträgt 0,24 %. Der Mannitanteil beträgt 0,05 %. Der Katalysator war nach einer Laufzeit von 1.600 Stunden noch unverändert wirksam.

### Beispiel 6

In einem Hochdruckrohr wie in Beispiel 1 wird bei einer Temperatur von 75°C und einem Wasserstoffdruck von 150 bar in gleicher Weise wie in Beispiel 1 eine ebenso große Menge einer 45 %igen wäßrigen Lösung von α-D-Glucopyranosido-1,6-fructose hydriert, die einen pH-Wert von 5,5 aufweist. Der Katalysator wurde durch Tablettierung einer pulverisierten Ni/Cr-Legierung gewonnen, die auch Al enthält. Die Legierung besitzt einen Cr-Gehalt von 4,1 % und einen Al-Gehalt von 5,4 %. Die Tabletten haben bei einer Zylinderhöhe von 5 mm und einem Durchmesser von 5 mm eine Druckfestigkeit von 158 N und eine innere Oberfläche von 58 m²/g. Das in einem Vakuum-Drehrohr gewonnene Gemisch von α-D-Glucopyranosido-1,6-mannit und α-D-Glucopyranosido-1,6-sorbit hat einen Reinheitsgrad von 99,2 %. Der Gehalt an nicht umgesetzter α-D-Glucopyranosido-1,6-fructose liegt bei 0,2 %. Der Sorbit-Gehalt beträgt 0,15 %. Mannit läßt sich nicht nachweisen. Der Katalysator war nach einer Laufzeit von 1.100 Stunden noch unvermindert wirksam.

## Patentansprüche

1. Verfahren zur Herstellung eines Gemisches von α-D-Glucopyranosido-1,6-mannit und α-D-Glucopyranosido-1,6-sorbit aus α-D-Glucopyranosido-1,6-fructose durch katalytische Hydrierung in wäßriger Lösung mit Wasserstoff unter erhöhtem Druck und bei erhöhter Temperatur, dadurch gekennzeichnet, daß man die Hydrierung kontinuierlich bei einem Wasserstoffdruck von 100 bis 400 bar und Temperaturen von 40 bis 80°C im Festbettverfahren über als Hydrierungskatalysatoren dienenden trägerfreien Formkörpern mit einer Druckfestigkeit von 20 bis 250 N und einer inneren Oberfläche von 10 bis 80 m²/g von (i) einem oder mehreren Elementen der Eisenuntergruppe der VIII. Nebengruppe des Periodensystems durchführt, die zusätzlich mit (ii) aktivierend wirkenden Elementen der VI. Nebengruppe legiert sind.

2. Verfahren nach Anspruch 1, dadurch gekennzeichnet, daß es sich bei den Formkörpern um solche aus verpreßten Metallpulvern handelt.

3. Verfahren nach Anspruch 1, dadurch gekennzeichnet, daß die Formkörper - bedingt durch das Preßverfahren - eine glatte Oberfläche aufweisen.

4. Verfahren nach Anspruch 1, dadurch gekennzeichnet, daß die Formkörper zylinderförmig oder kugelförmig sind und Durchmesser von 3 bis 7 mm besitzen.

5. Verfahren nach Anspruch 1, dadurch gekennzeichnet, daß man die Hydrierung der α-D-Glucopyranosido-1,6-fructose in 45- bis 60 gew.-%iger wäßriger Lösung bei einem pH-Wert von 3,5 bis 10,5 durchführt.

## Claims

1. Process for the preparation of a mixture of α-D-glucopyranosido-1,6-mannitol and α-D-glucopyranosido-1,6-sorbitol from α-D-glucopyranosido-1,6-fructose by catalytic hydrogenation in aqueous solution with hydrogen at elevated pressure and elevated temperature, characterized in that the hydrogenation is carried out continuously at a hydrogen pressure of 100 to 400 bar, and temperatures of 40 to 80°C, in the fixed-bed process over support-free shaped bodies serving as hydrogenation catalysts and having a compressive strength of 20 to 250 N, and an internal surface area of 10 to 80 m²/g of (i) one or more elements of the iron sub group of sub group VIII of the Periodic Table of the Elements which are additionally alloyed with (ii) elements of sub group VI having activating activity.

2. Process according to Claim 1, characterized in that the shaped bodies are those composed of pressed metal powders.

3. Process according to Claim 1, characterized in that the shaped bodies - because of the pressing process - have a smooth surface.

4. Process according to Claim 1, characterized in that the shaped bodies are cylindrical or spherical and have diameters from 3 to 7 mm.

5. Process according to Claim 1, characterized in that the hydrogenation of the α-D-glucopyranosido-1,6-fructose is carried out in 45 to 60 % strength by weight aqueous solution at a pH of 3.5 to 10.5.

## Revendications

1. Procédé pour la préparation d'un mélange d'α-D-glucopyrannosido-1,6-mannitol et d'α-D-glucopyrannosido-1,6-sorbitol à partir de l'α-D-glucopyrannosido-1,6-fructose par hydrogénation catalytique en solution aqueuse à pression et température élevées, caractérisé en ce que l'on réalise l'hydrogénation en continu sous une pression d'hydrogène de 100 à 400 bar et à des températures de 40 à 80°C par la technique à lit fixe sur des corps moulés sans support servant de catalyseurs d'hydrogénation et ayant une résistance à la compression de 20 à 250 N et une surface interne de 10 à 80 m²/g et qui consistent en (i) un ou plusieurs éléments du groupe du fer du sous-groupe VIII de la Classification Périodique, alliés en outre avec (ii) des éléments activants du sous-groupe VI.

2. Procédé selon la revendication 1, caractérisé en ce que les corps moulés consistent en poudres métalliques comprimées.

3. Procédé selon la revendication 1, caractérisé en ce que les corps moulés - en raison de l'opération de compression - ont une surface lisse.

4. Procédé selon la revendication 1, caractérisé en ce que les corps moulés ont la forme de cylindres ou de sphérules à des diamètres de 3 à 7 mm.

5. Procédé selon la revendication 1, caractérisé en ce que l'on hydrogène l'α-D-glucopyrannosido-1,6-fructose à l'état de solution aqueuse à une concentration de 45 à 60 % en poids et à un pH de 3,5 à 10,5.
